# EUROPÄISCHE PATENTANMELDUNG

(11) **EP 1 038 549 A2**
(43) Veröffentlichungstag der Anmeldung: **27.09.2000**
(21) Anmeldenummer: 99126237.9
(22) Anmeldetag: 30.12.1999
(51) Int. Cl.: A61N 2/02

(54) **Medizinisches Behandlungsgerät mit magnetischen Wellen**

(30) Priorität: 30.12.1998 DE 29823196 U
(71) Anmelder: Bartik, Wolfgang, 81243 München (DE); Buschky, Rudolf, 74861 Neudenau (DE)
(72) Erfinder: Bartik, Wolfgang, 81243 München (DE); Buschky, Rudolf, 74861 Neudenau (DE)

(57) **Zusammenfassung**

Die vorliegende Erfindung betrifft ein medizinisches Behandlungsgerät mit offenen Flachspulen (Induktionsspulen) zur Erzeugung pulsierender Magnetfelder zur Beeinflussung der magno - energetischen Vorgänge in lebenden Organismen (Menschen, Tiere, Pflanzen), hier speziell Stoffwechselbeeinflussung / Hormonlagebeeinflussung !

Die Aufgabe besteht in der Applikation/Anwendung der energetischen Wellen dergestalt, daß der zu behandelnde Organismus/Stoffwechsel
a) zid-u.punktgenau
b) polaritätsmäßig /unipolar/bipolar
c) Frequenz-pulsmäßig
d) intensitätsmäßig
so erfaßt werden kann, daß eine optimale "Wirbelstromerzeugung" im Organismus hervorgerufen wird. Die therapeutische Wirkung entsteht dabei durch die sich reflektorisch in Folge aufbauende "Resonanz" im behandeltem Körper! (Resonanz-Meßabfrage) Die vorliegende Erfindung erfüllt diese Bedingungen mit a) rotierende Spulen; b) durch die Drehbewegung mögliche Polaritätsumschaltungen; durch die Drehbewegung steuerbare Pulsationsgabe; d) durch Kontaktgabe Focusierung/Bündelung des Magnetflusses u. Intensitätssteuerung möglich. Um auch die unmittelbaren "Randbereiche" zu erfassen, wird der bewegliche /steuerbar rotierende Spulenträger in Form einer "Taumelscheibe" ausgerührt! Die entsprechenden "Taumelbewegungen" sind ebenfalls steuerbar.Die "Rotation kann elektromechanisch als auch rein elektronisch erfolgen; die "Taumelsteuerung erfolgt rein elektromechanisch!

## Beschreibung

### TECHNISCHES GEBIET

Die vorliegende Erfindung betrifft ein medizinisches Behandlungsgerät mit mehreren flachen Induktionsspulen zur Erzeugung pulsierender Magnetfelder zur Beeinflussung der magneto-energetischen Vorgänge im lebenden Menschen und Tiere mit einer Halteeinrichtung, an der die Induktionsspulen elektrisch isoliert vorhanden sind.

Derartige Behandlungsgeräte dienen zum magnetischen, therapeutischen Behandeln von Lebewesen. Ein Patient setzt das Behandlungsgerät an und begibt sich somit in unmittelbare Nähe des Geräts, dessen Induktionsspulen dann von einem Strom durchflossen werden. Der Patient wird dann von den von den Induktionsspulen ausgehenden Magnetfelder durchströmt und dadurch wird der therapeutische Behandlungseffekt erzielt.

### STAND DER TECHNIK

Aus der deutschen Offenlegungsschrift 31 28 263 ist eine Vorrichtung zur Beeinflussung magneto-energetischer Vorgänge im Menschenorganismus bekannt. Dabei werden gegenüberliegende Behandlungsköpfe eingesetzt, die mit mehreren paarweise betriebenen Induktionsspulen ausgestattet sind.

Aus der deutschen Offenlegungsschrift 38 28 43 ist ein medizinisches Behandlungsgerät bekannt, das als flexibles, flaches Kissen mit mehreren Induktionsspulen ausgebildet ist, die mit pulsierenden Gleichstrom betrieben werden.

Schließlich ist aus dem deutschen Patent 40 04 682 eine Magnetdecke zur Ganzkörperbehandlung beschrieben, bei der die Induktionsspulen zu Spulengruppen zusammengefaßt sind und unterschiedliche Spulengruppen zeitlich versetzt vom Erregerstrom angesteuert werden, so daß sich ein "wanderndes" Magnetfeld einstellt.

### DARSTELLUNG DER ERFINDUNG

Der vorliegenden Erfindung liegt die Aufgabe beziehungsweise das technische Problem zugrunde, ein medizinisches Behandlungsgerät anzugeben , das einen einfachen konstruktiven Aufbau besitzt, eine gute therapeutische Wirkung erzielt und das variabel einsetzbar ist.

Das erfindungsgemäße Behandlungsgerät ist durch die Merkmale des unabhängigen Anspruchs 1 gegeben. Vorteilhafte Ausgestaltungen und Weiterbildungen sind Gegenstand der abhängigen Ansprüche.

Das medizinische Behandlungsgerät zeichnet sich demgemäß dadurch aus, daß die Halteeinheit um eine Drehachse rotierbar mittels eines Antriebsaggregats über eine ortsfeste Lagereinrichtung ausgebildet ist, wobei die Drehachse im wesentlichen senkrecht zur Ebene der flachen Induktionsspulen angeordnet ist, die Halteeinrichtung zwei Kontakteinheiten für jede Induktionsspule oder für mehrere elektrisch leitende miteinander verbundene Induktionsspulen aufweist, die Lagereinrichtung zumindest zwei elektrisch isoliert vorhandene Kontaktflächen aufweist, an die der Pluspol beziehungsweise Minuspol einer Stromquelle angeschlossen ist, und die Kontakteinheiten der Halteeinrichtung während der Rotation zumindest zeitweise mit den Kontaktflächen der Lagereinrichtung in Kontakt kommt, so daß ein Strom durch die Induktionsspulen fließt und dadurch Magnetfelder aufgebaut werden.

Eine besonders bevorzugte Ausgestaltung mit konstruktiv einfachen Aufbau zeichnet sich dadurch aus, daß die Kontakteinheiten unterseitig an der Halteeinrichtung vorhanden sind.

Eine gute therapeutische Wirkung läßt sich mit einer vorteilhaften Ausgestaltung erzielen, die sich dadurch auszeichnet, daß sämtliche Induktionsspulen elektrisch leitend untereinander verbunden sind, und an insgesamt zwei Kontakteinheiten angeschlossen sind, wobei die Induktionsspulen parallel oder in Reihe geschaltet sein können.

Eine vorteilhafte Ausgestaltung zeichnet sich dadurch aus daß die Kontakteinheiten und die Kontaktflächen so angeordnet und geschaltet sind, daß sich bei einer Umdrehung der Halteeinrichtung die Polarisierung des Magnetfelds in der Induktionsspule mehrmals ändert.

Eine alternative Weiterbildung zeichnet sich dadurch aus daß die Kontakteinheiten und die Kontaktflächen so angeordnet und geschaltet sind, daß sich bei Drehung der Halteeinrichtung die Polarisierung der Magnetfelder von im Umfangsrichtung aufeinanderfolgender Induktionsspulen alternierend ändert.

Schließlich zeichnet sich eine weitere Alternativausbildung dadurch aus, daß die Kontakteinheiten und die Kontaktflächen so angeordnet und geschaltet sind, daß sich bei Drehung der Halteeinrichtung die Polarisierung sämtlicher Magnetfelder der Induktionsspulen alternierend ändert.

Die Spannung ist bevorzugt als Gleichspannung im Bereich zwischen 6 bis 12 Volt ausgebildet.

Bei einer besonders vorteilhaften Ausgestaltung ist eine Steuereinrichtung vorhanden, mittels derer die Rotationsgeschwindigkeit der Halteeinrichtung gesteuert werden kann.

Weitere Ausführungsformen und Vorteile der Erfindung ergeben sich durch die in den Ansprüchen ferner aufgeführten Merkmalen sowie durch das nachstehend angegebene Ausführungsbeispiel. Die Merkmale der Ansprüche können in beliebiger Weise miteinander kombiniert werden, insoweit sie sich nicht offensichtlich gegenseitig ausschließen

### KURZE BESCHREIBUNG DER ZEICHNUNG

Die Erfindung sowie vorteilhafte Ausführungsformen und Weiterbildungen derselben werden im folgenden anhand dem in der Zeichnung dargestellten Beispiel näher beschrieben und erläutert. Die der Beschreibung und der Zeichnung zu entnehmende Merkmale können einzeln für sich oder zu mehreren in beliebiger Kombination erfindungsgemäß angewandt werden. Es zeigen:
- Fig. 1: schematische Darstellung von zwei an eine Tranformatoreinheit angeschlossenen medizinischen Behandlungsgeräten,
- Fig. 2: stark schematisierte Darstellung einer ersten Ausführungsform eines medizinischen Behandlungsgeräts in auseinander gezogenem Zustand mit Kontakteinheiten und Kontaktflächen,
- Fig. 3: stark schematisierte Darstellung eines zweiten Ausführungsform eines medizinischen Behandlungsgeräts in auseinander gezogenem Zustand mit Kontakteinheiten und Kontaktflächen,
- Fig.4: stark schematisierte Darstellung eines dritten Ausführungsform eines medizinischen Behandlungsgeräts in auseinander gezogenem Zustand mit Kontakteinheiten und Kontaktflächen,
- Fig. 5: stark schematisierte Detaildarstellung einer weiteren Ausführungsform der Kontaktflächen,
- Fig. 6: stark schematisierte Detaildarstellung einer weiteren Ausgestaltung der Kontaktflächen und
- Fig. 7: schematisierte Schnitt durch ein Behandlungsgerät gemäß Fig. 1.

### WEGE ZUM AUSFÜHREN DER ERFINDUNG

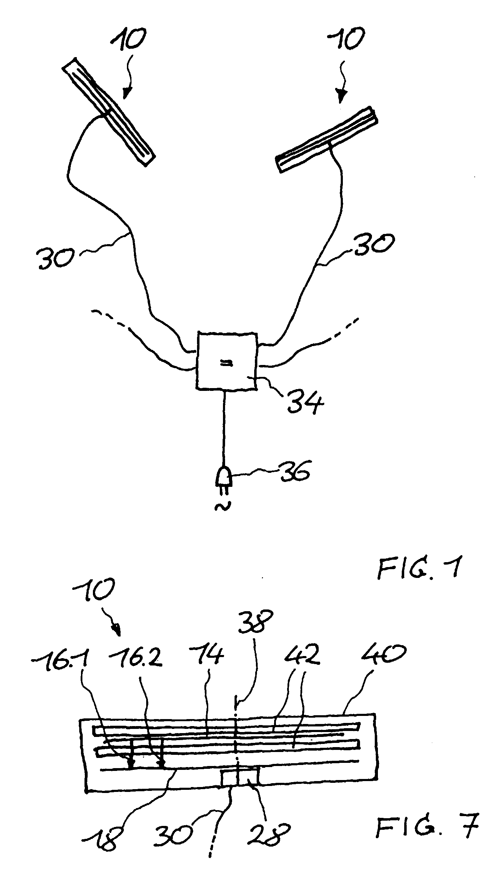

In Fig.1 ist schematisch ein Behandlungsgerät 10 dargestellt, das über eine Leitung 30 an eine Transformator- einheit 34 elektrisch leitend angeschlossen ist, wobei die Transformatoreinheit 34 über ein Stecker 36 an ein Stromnetz anschließbar ist.

Die Transformatoreinheit 34 transformiert den üblicherweise im Stromkreis vorhandenen Wechselstrom in eine Gleichspannung von beispielsweise 12 Volt. Mit dieser Gleichspannung werden Induktionsspulen 12 beaufschlagt, so daß sich beim Fließen eines Stromes ein Magnetfeld aufbaut, daß zu therapeutischen Zwecken eingesetzt wird.

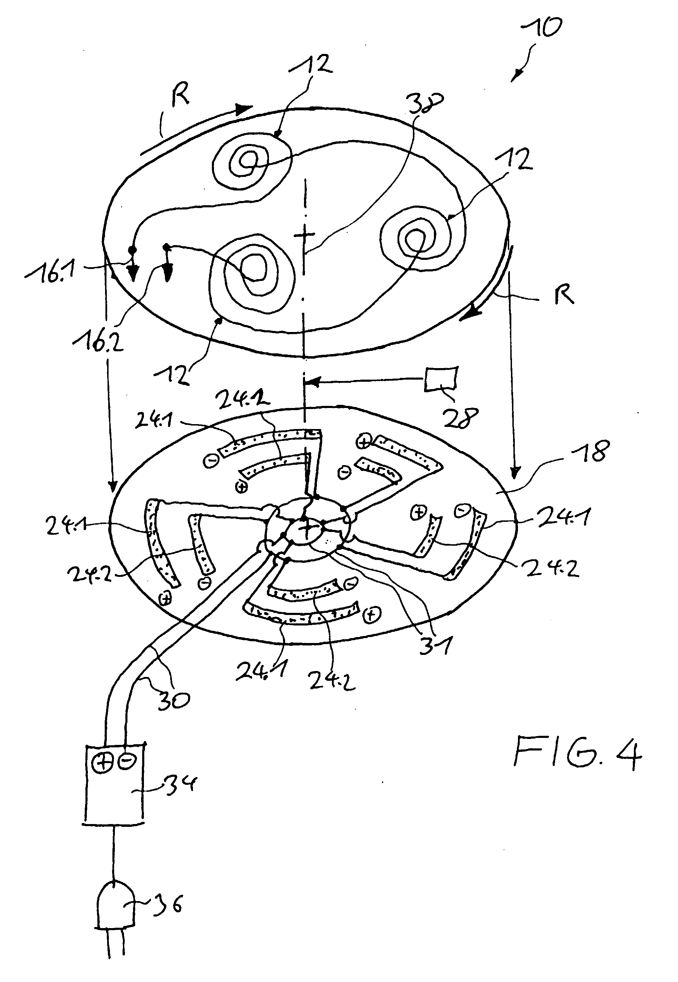

In Fig. 7 ist ein schematischer Schnitt durch ein Behandlungsgerät 10 dargestellt. Es ist eine als Scheibe ausgebildete Halteeinrichtung 14 vorhanden, auf der Induktionsspulen angeordnet sind, wobei die Halteeinrichtung 14 beziehungsweise die Induktionsspulen von Isolierfolien 42 umgeben sind. Die als Kreisscheibe ausgebildete Halteeinrichtung 14 ist um eine Drehachse 38 drehbar gelagert, wobei die Drehachse 38 durch den Mittelpunkt der Kreisscheibe verläuft und senkrecht zur Kreisebene steht. Die Rotation wird durch ein in Fig. 7 schematisch dargestelltes Antriebsaggregat 28 bewirkt, wobei die Rotationsgeschwindigkeit über eine nicht dargestellte Steuereinrichtung gesteuert werden kann. Schließlich ist noch ein Gehäuse 40 vorhanden, daß das Behandlungsgerät 10 nach außen hin umschließt.

Unterseitig sind an der Halteeinrichtung 14 zwei Kontakteinheiten 16.1 und 16.2 vorhanden. Die Kontakteinheiten 16.1, 16.2 sind soweit nach unten geführt, bis sie auf eine ortsfeste unterhalb der Halteeinrichtung 14 vorhandene Lagereinrichtung 18 treffen, die ebenfalls als Kreisscheibe ausgebildet ist. Die Kontakteinheiten 16.1 und 16.2 sind elektrisch leitend an die Induktionsspulen angeschlossen und kommen infolge der Rotation der Halteeinrichtung 14 mit auf der Lagereinrichtung 18 vorhandenen Kontaktflächen in elektrischen Kontakt.

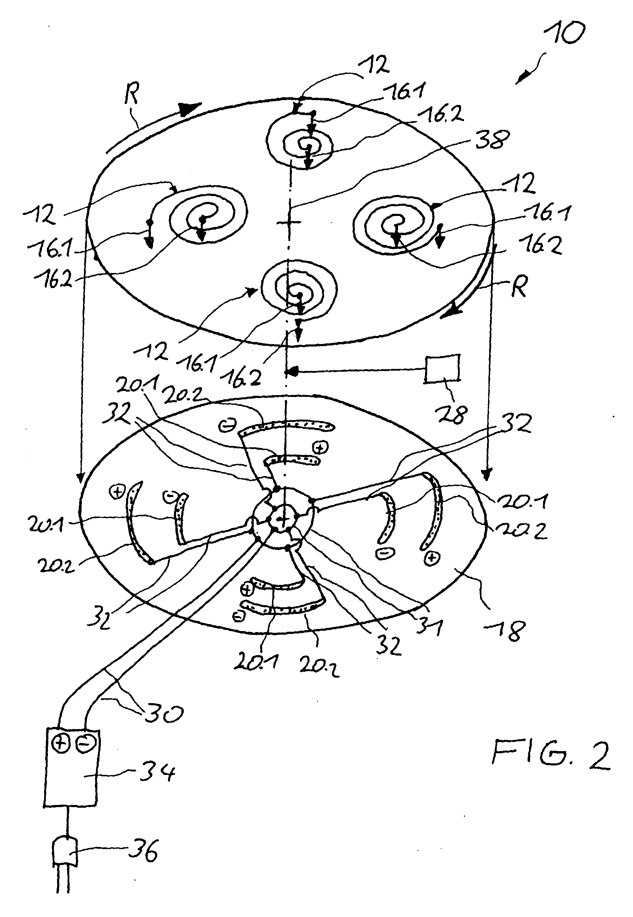

Schematisiert dargestellte Ausführungsbeispiele sind den Figuren 2 bis 6 zu entnehmen.

Gemäß Fig. 2 sind in einem ersten Ausführungsbeispiel auf der Halteeinrichtung 14 insgesamt in Umfangsrichtung um jeweils 90° versetzt 4 flache Induktionsspulen 12 angeordnet, von denen jede an zwei Kontakteinheiten 16.1 und 16.2 angeschlossen ist. Aus Übersichtlichkeitsgründen ist in Fig. 2 eine auseinander gezogene Darstellung ohne Gehäuse und Isolierfolie gewählt. Die Halteeinrichtung 14 kann um ihre Drehachse 38 über ein schematisch dargestelltes Antriebsaggregat 28 in Rotation (R) versetzt werden.

Unterhalb der Halteeinrichtung 14 ist die Lagereinrichtung 18 ortsfest vorhanden, die entsprechend der radialen Anordnung der Kontakteinheiten 16.1 und 16.2 entsprechende Kontaktflächen 20.1 beziehungsweise 20.2 aufweist.

Die Kontaktflächen 20.1, 20.2 besitzen den selben Abstand zur Drehachse 38 wie die Kontakteinheiten 16.1 und 16.2. Insgesamt sind die Kontaktflächen 20.1, 20.2 in Umfangsrichtung um 90° verteilt als jeweils vier teilkreisförmige Kontaktflächenbereiche ausgebildet. Im Zentrum der Lagereinrichtung 18 befinden sich zwei Ringleitungen 31, an die die Stromführungsleitung 30 angeschlossen ist. Die Kontaktflächen 16.1 und 16.2 selbst sind über Leiterbahnen 32 an diese Ringleitungen 31 angeschlossen und zwar derart, daß die Polanschlüsse in Umfangsrichtung aufeinanderfolgender Kontaktflächen jeweils vertauscht sind.

Betrachtet man eine Induktionsspule 12 während einer Umdrehung, so wird gemäß dem dargestellten Ausführungsbeispiel insgesamt viermal ein Magnetfeld erzeugt, wobei die Polarisierung des Magnetfelds alternierend immer wieder umschlägt. Dieser Vorgang betrifft sämtliche vier auf der Halteeinrichtung 14 angeordnete Induktionsspulen 12.

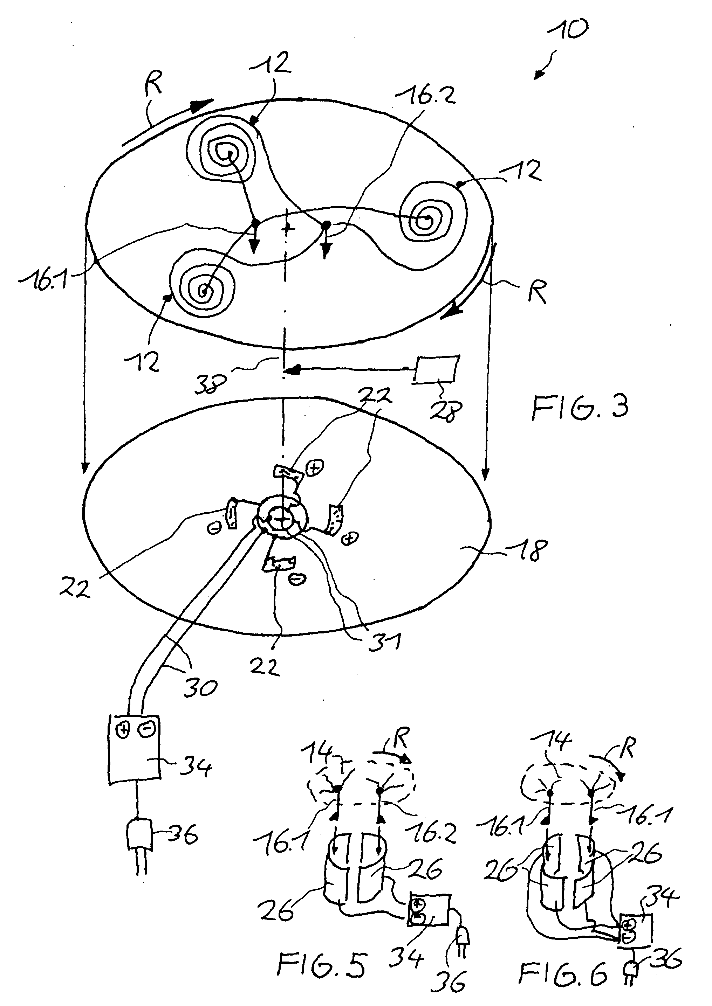

In Fig. 3 ist ein weiteres Ausführungsbeispiel schematisch dargestellt. Insgesamt sind drei in Umfangsrichtung um 120° versetzt angeordnete Induktionsspulen 12 vorhanden, die elektrisch leitend miteinander verbunden sind und auf zwei bezüglich der Drehachse 38 im Inneren gegenüberliegenden Kontakteinheiten 16.1 und 16.2 geschaltet sind. Insgesamt sind auf der Lagereinrichtung 18 vier in Umfangsrichtung um 90° versetzte Kontaktflächen 22 vorhanden, die den selben Abstand zur Drehachse 38 aufweisen wie die Kontakteinheiten 16. 1 beziehungsweise 16.2. Über die Ringleitungen 31 sind die Kontaktflächen 22 so an die Transfomatoreinheit 34 angeschlossen, daß an zwei von ihnen der Minuspol und an zwei der Pluspol der Transformatoreinheit 34 ansteht.

Bei einer Drehung der Halteeinrichtung 14 wird zunächst kurzfristig in allen drei Induktionsspulen 12 jeweils ein Magnetfeld mit gleicher Polung aufgebaut. Nach einer halben Umdrehung wird zum zweiten mal ein Magnetfeld aufgebaut, doch mit umgekehrter Polarisierung.

Das Ausführungsbeispiel gemäß Fig. 4 ähnelt hinsichtlich seiner Kontaktflächen 24.1 beziehungsweise 24.2 dem Ausführungsbeispiel gemäß Fig. 2, jedoch sind nur drei Induktionsspulen 12 vorhanden, die elektrisch leitend in Reihe geschaltet sind. Somit sind auf der Halteeinrichtung 14 nur zwei Kontakteinheiten 16.1 beziehungsweise 16.2 vorhanden. Auch hier wird ein pulsierendes Magnetfeld erzeugt infolge Rotation der Halteeinrichtung 14 mit den Induktionsspulen 12, wobei sämtliche drei Induktionsspulen 12 immer alternierend jeweils am Magnetfeld mit gleicher Polarisierung aufbauen.

Die in den Ausführungsbeispielen dargestellten Kontaktflächen stellen lediglich eine kleine Auswahl an konstruktiven Möglichkeiten dar. Insbesondere kann die Frequenz der Erzeugung von Magnetfelder durch geeignete Wahl der Geometrie der Kontaktflächen bestimmt werden, wobei diese Frequenz auch von der Rotationsgeschwindigkeit der Halteeinrichtung beeinflußbar ist.

In Fig. 5 ist im Detail eine anders geartete Ausbildung der Kontaktflächen dargestellt. Die Kontaktflächen 26 sind als zylindrische Halbschalen ausgebildet, die gegeneinander elektrisch isoliert sind und an denen die Kontakteinheiten 16.1 beziehungsweise 16.2 (in Fig. 5 auseinander gezogen dargestellt) gegenseitig entlang schleifen, das heißt die Kontakteinheiten 16.1, 16.2 können als Schleifkontakte ausgebildet sein. Eine Halbschale liegt am Pluspol, die andere Halbschale liegt am Minuspol an.

In Fig. 6 sind die Kontaktflächen 20 als viertelzylindrische Schalen ausgebildet, die untereinander elektrisch isoliert vorhanden sind und die alternierend mit dem Plusbeziehungsweise mit dem Minuspol der Transformatoreinheit 34 verbunden sind.

## Patentansprüche

1. Medizinisches Behandlungsgerät (10) mit mehreren flachen Induktionsspulen (12) zur Erzeugung pulsierender Magnetfelder zur Beeinflussung der magneto-energetischen Vorgänge in lebenden Menschen und Tieren mit einer Halteeinrichtung (14), an der die Induktionsspulen (12) elektrisch isoliert vorhanden sind,
**dadurch gekennzeichnet, daß**
- die Halteeinheit (14) um eine Drehachse (38) rotierbar mittels eines Antreibsaggregats (28) über einer ortsfesten Lagereinrichtung (18) ausgebildet ist, wobei die Drehachse (38) im wesentlichen senkrecht zur Ebene der flachen Induktionsspulen (12) angeordnet ist,
- die Halteeinrichtung (14) zwei Kontakteinheiten (16.1, 16.2) für jede Induktionsspule (12) oder für mehrere elektrisch leitende miteinander verbundene Induktionsspulen (12) aufweist,
- die Lagereinrichtung (18) zumindest zwei elektrisch isoliert vorhandene Kontaktflächen (20.1, 20.2; 22; 24.1, 24.2; 26) aufweist, an die der Pluspol beziehungsweise Minuspol einer Stromquelle angeschlossen ist, und
- die Kontakteinheiten (16.1, 16.2) der Halteeinrichtung (14) während der Rotation zumindest zeitweise mit den Kontaktflächen (20.1, 20.2; 22; 24.1, 24.2; 26) der Lagereinrichtung (18) in Kontakt kommen, so daß ein Strom durch die Induktionsspulen (12) fließt und dadurch Magnetfelder aufgebaut werden.

2. Behandlungsgerät nach Anspruch 1,
**dadurch gekennzeichnet, daß**
- die Kontakteinheiten (16.1, 16.2) unterseitig an der Halteeinrichtung (14) vorhanden sind.

3. Behandlungsgerät nach Anspruch 1 oder 2,
**dadurch gekennzeichnet, daß**
- sämtliche Induktionsspulen (12) elektrisch leitend untereinander verbunden sind und an insgesamt zwei Kontakteinheiten (16.1, 16.2) angeschlossen sind.

4. Behandlungsgerät nach Anspruch 3,
**dadurch gekennzeichnet, daß**
- die Induktionsspulen (12) parallel geschaltet sind.

5. Behandlungsgerät nach einem oder mehreren Ansprüche 1 bis 3,
**dadurch gekennzeichnet, daß**
- die Induktionsspulen (12) in Reihe geschaltet sind.

6. Behandlungsgerät nach einem oder mehreren der vorstehenden Ansprüche,
**dadurch gekennzeichnet, daß**
- die Kontakteinheiten (16.1, 16.2) und die Kontaktflächen (20.1, 20.2; 22; 24.1, 24.2; 26) so angeordnet und geschaltet sind, daß sich bei einer Umdrehung der Halteeinrichtung (14) die Polarisierung des Magnetfelds in einer Induktionsspule (12) mehrmals ändert.

7. Behandlungsgerät nach einem oder mehreren der vorstehenden Ansprüche,
**dadurch gekennzeichnet, daß**
- die Kontakteinheiten (16.1, 16.2) und die Kontaktflächen (20.1, 20.2; 22; 24.1, 24.2; 26) so angeordnet und geschaltet sind, daß sich bei Drehung der Halteeinrichtung (14) die Polarisierung der Magnetfelder in Umfangsrichtung aufeinanderfolgender Induktionsspulen (12) alternierend ändert.

8. Behandlungsgerät nach einem oder mehreren der Ansprüche 1 bis 6,
**dadurch gekennzeichnet, daß**
- die Kontakteinheiten (16.1, 16.2) und die Kontaktflächen (20.1, 20.2; 22; 24.1, 24.2; 26) so angeordnet und geschaltet sind, daß sich bei Drehung der Halteeinrichtung (14) die Polarisierung sämtlicher Magnetfelder der Induktionsspulen alternierend ändert.

9. Behandlungsgerät nach einem oder mehreren der vorstehenden Ansprüche,
**dadurch gekennzeichnet, daß**
- die an den Kontakteinheiten (16.1, 16.2) anliegende Spannung als Gleichspannung ausgebildet ist, insbesondere im Bereiche zwischen 6 V (Volt) bis 12 V (Volt).

10. Behandlungsgerät nach einem der vorstehenden Ansprüche,
**dadurch gekennzeichnet, daß**
- die Kontakteinheiten (16.1, 16.2) und die Kontaktflächen (20.1, 20.2; 22; 24.1, 24.2; 26) so angeordnet sind beziehungsweise die Rotationsgeschwindigkeit der Halteeinrichtung (14) so gewählt ist, daß die Frequenz, mit der die Polarisierung jedes Magnetfelds einer Induktionsspule (12) wechselt im Bereich zwischen 2 bis 30 Hz (Hertz) liegt.

11. Behandlungsgerät nach einem oder mehreren der vorstehenden Anspürche,
**dadurch gekennzeichnet, daß**
- eine Steuereinrichtung vorhanden ist, mittels derer die Rotationsgeschwindigkeit der Halteeinrichtung (14) einstellbar ist.

12. Behandlungsgerät nach einem oder mehreren der vorstehenden Ansprüche,
**dadurch gekennzeichnet, daß**
- drei oder vier Induktionsspulen (12) auf der Halteeinrichtung angeordnet sind.
